# EUROPEAN PATENT SPECIFICATION

(11) **EP 2 775 836 B1**
(45) Date of publication and mention of the grant of the patent: **12.09.2018**
(21) Application number: 11875217.9
(22) Date of filing: 04.11.2011
(51) Int. Cl.: A61P 19/06

(54) **METHODS FOR TREATING GOUT FLARES**
VERFAHREN ZUR BEHANDLUNG VON GICHTFÄRBUNGEN
MÉTHODES PERMETTANT DE TRAITER LES ÉRYTHÈMES DE LA GOUTTE

(43) Date of publication of application: 17.09.2014
(73) Proprietor: CymaBay Therapeutics, Inc., Newark, CA 94560 (US); DiaTex, Inc., San Antonio, TX 78231 (US)
(72) Inventor: LAVAN, Brian Edward, NEWARK CA 94560 (US); SAHA, Gopal Chandra, NEWARK CA 94560 (US); ROBERTS, Brian K., NEWARK CA 94560 (US); MCWHERTER, Charles A., NEWARK CA 94560 (US)
(74) Representative: Wills, Andrew Jonathan
(86) International application number: PCT/US2011/059433
(87) International publication number: WO 2013/066353

(56) References cited:
- US-A1- 2011 268 801
- US-B1- 6 613 802
- BLUHM G B ET AL: "A double blind study comparing halofenate with probenecid in gout", ARTHRITIS & RHEUMATISM, WILEY, US, vol. 18, no. 4, 1 January 1975 (1975-01-01), pages 388-389, XP009183634, ISSN: 0004-3591
- ANONYMOUS: "METABOLEX INITIATES PHASE 2 TRIAL OF ARHALOFENATE Potential Best-in-Class Uricosuric Agent for the Treatment of Gout", INTERNET CITATION, 19 May 2011 (2011-05-19), pages 1-2, XP002682224, Retrieved from the Internet: URL:http://www.metabolex.com/news/may19201 1.html [retrieved on 2012-02-11]
- Schlesinger N: "Management of acute and chronic gouty arthritis: present state-of-the-art. - PubMed - NCBI", , 1 January 2004 (2004-01-01), XP055181670, Retrieved from the Internet: URL:http://www.ncbi.nlm.nih.gov/pubmed/154 81999 [retrieved on 2015-04-08]

## Description

### BACKGROUND

This application relates to the treatment, including the prevention, of gout flares.

Schlesinger N. (Drugs, 2004, 64(21):2399-416) discloses three separate stages in gout management. This publication emphasizes the distinction between therapy for reducing acute inflammation in acute gout and therapy to manage hyperuricaemia in patients with chronic gouty arthritis. NSAIDs are said to be the preferred treatment in acute gout attacks, and nonpharmacological treatments are said to be useful. Various drugs are proposed for a longstanding reduction in serum uric acid to manage chronic gout.

### SUMMARY

This application describes methods of treating a gout flare experienced by a subject comprising administering to the subject a compound of Formula (I) 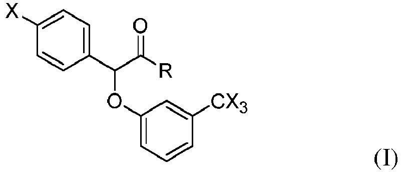 wherein R is selected from the group consisting of a hydroxy, lower aralkoxy, di-lower alkylamino-lower alkoxy, lower alkanamido-lower alkoxy, benzamido-lower alkoxy, ureido-lower alkoxy, N'-lower alkyl-ureido-lower alkoxy, carbamoyl-lower alkoxy, halophenoxy substituted lower alkoxy, carbamoyl substituted phenoxy, carbonyl-lower alkylamino, N,N-di-lower alkylamino-lower alkylamino, halo substituted lower alkylamino, hydroxy substituted lower alkylamino, lower alkanolyloxy substituted lower alkylamino, ureido, and lower alkoxycarbonylamino; and each X is independently a halogen, or a pharmaceutically acceptable salt thereof.

Also disclosed are methods of reducing the number, duration, frequency or intensity of gout flares experienced by a subject comprising administering a compound of Formula (I) or a pharmaceutically acceptable salt thereof to the subject. Also disclosed is the treatment of hyperuricemia in a subject with gout comprising administering to a subject in need thereof a compound of Formula (I), wherein the dose, frequency, and duration of administration are effective to reduce the number, duration, frequency, or intensity of gout flares experienced by the subject during the duration. Also disclosed are methods of providing to a subject (-)-halofenic acid with an intraday peak-to-trough ratio of about 2.0 or less. Further aspects are provided below. The invention is defined in claim 1. Preferred features are defined in the dependent claims.

Uric acid lowering agents such as allopurinol and febuxostat generally increase the number, duration, frequency, or intensity of gout flares upon initiation of therapy, and this exacerbation may last for several weeks to months following initiation of such therapy. Uric acid lowering agents often require a dose titration strategy in which the dose is progressively increased to the therapeutic dose in order to minimize the number, duration, frequency, or intensity of flares. Flare treatment or prophylaxis with an additional therapeutic agent such as a non-steroidal anti-inflammatory agent (NSAID) or colchicine is often recommended during this period. During longer term maintenance use of urate lowering therapy, flares can also be precipitated by fluctuations in uric acid levels caused by non-adherance with prescribing instructions. Advantages of the current methods include decreasing the number, duration, frequency, or intensity of flares experienced by the patient (e.g. during initiation or maintenance of therapy for uric acid lowering), decreasing the need for dose titration, and reducing the amount or duration of additional anti-flare medicaments.

### BRIEF DESCRIPTION OF THE DRAWINGS

**FIG. 1** is a graph showing the mean trough plasma concentration values of (-)-halofenic acid during and following a 30-day dosing schedule of daily oral administration of 400 mg of arhalofenate.
**FIG. 2** is a graph showing the mean and standard deviation (SD) of (-)-halofenic acid plasma concentrations at Day 15 and Day 30 following daily oral administration of 400 mg of arhalofenate in 20 human subjects.
**FIG. 3** is a graph showing reduction in serum uric acid in subjects over time following once daily dosing with arhalofenate.
**FIG. 4** is a graph showing the effect of (-)-halofenic acid on lowering the messenger RNA encoding the pro-inflammatory cytokine IL-1β in lipopolysaccharide (LPS) stimulated primary mouse macrophages.
**FIG. 5** is a graph showing the effect of (-)-halofenic acid on lowering the secretion of the pro-inflammatory cytokine IL-1β from lipopolysaccharide stimulated primary mouse macrophages.
**FIG. 6** is a chart showing mean lowering of high sensitivity C-reactive protein (hs-CRP) levels in human subjects after treatment with (-)-halofenate 600 mg.
**FIG. 7** is a chart showing mean percent lowering of high sensitivity C-reactive protein (hs-CRP) levels in human subjects after treatment with (-)-halofenate 600 mg.

### DETAILED DESCRIPTION

As utilized in accordance with the present disclosure, the following terms, unless otherwise indicated, shall be understood to have the following meanings:

"About" when qualifying a number, refers to a range of plus or minus ten percent of that value or number, unless indicated otherwise. Without limiting the application of the doctrine of equivalents as to the scope of the claims, each number should be construed in light of such factors as the number of reported significant digits and the manner or method (e.g. instrumentation, sample preparation, etc.) used to obtain that number.

"Administering" or "administration" refers to the act of giving a drug, prodrug, or therapeutic agent to a subject. Exemplary routes of administration are discussed below.

"Acute gout" refers to gout present in a subject with at least one gouty symptom (e.g., podagra or other gouty arthritis, gout flare, gouty attack).

"Chronic gout" refers to gout present in a subject having recurrent or prolonged gout flares, tophus formation, chronic inflammatory arthritis, or joint deterioration associated with gout, and includes the periods following recovery from acute gout and between acute gout attacks (i.e. intercritical gout).

"Composition" or, interchangeably, "formulation" refers to a preparation that contains a mixture of various excipients and key ingredients that provide a relatively stable, desirable, and useful form of a compound or drug.

The prefixes "d" and "l" or (+) and (-) are employed to designate the sign of rotation of plane-polarized light by the compound, with (+) or d- meaning that the compound is "dextrorotatory" and with (-) or 1- meaning that the compound is "levorotatory". For a given chemical structure, these isomers or "optical isomers" are identical except that they are mirror images of one another. In describing an optically active compound, the prefixes R and S are used to denote the absolute configuration of the molecule about its chiral center(s). There is no correlation between the nomenclature for the absolute stereochemistry and for the rotation of an enantiomer (i.e., the R- isomer can also be the 1- isomer). A specific optical isomer can also be referred to as an "enantiomer," and a mixture of such isomers is often called an "enantiomeric" or "racemic" mixture. *See, e.g.,* A. Streitwiesser & C.H. Heathcock, INTRODUCTION TO ORGANIC CHEMISTRY, 2nd Edition, Chapter 7 (MacMillan Publishing Co., U.S.A. 1981). The optical rotation [α]_{D} of (-)-halofenate was measured in methyl alcohol.

"Elevated serum uric acid level" refers to a serum uric acid level greater than normal and, in patients with gout, generally refers to a serum uric acid level greater than or equal to about 6 mg/dL. In some instances, elevated serum uric acid levels are above the mean level in a given population, such as those of a particular gender or age.

"Effective amount" refers to an amount required (i) at least partly to attain the desired response in a subject; (ii) to delay or to prevent the onset of a particular condition being treated in a subject; or (iii) or to inhibit or to prevent the progression of a particular condition being treated in a subject. The effective amount for a particular subject varies depending upon the health and physical condition of the subject to be treated, the taxonomic group of individual to be treated, the degree of protection desired, the formulation of the composition, the assessment of the medical situation, and other relevant factors. It is expected that the amount will fall in a relatively broad range that can be determined through routine trials.

"First urate-lowering agent" refers to a compound of any of Formulae (I), (II), (III), or (IV) or a therapeutically acceptable salt or prodrug thereof. For clarity, this term implies no temporal aspect or relationship, e.g. to a second urate-lowering agent.

"Flare" or "gout flare" refers to a symptom of gout associated with a sudden onset of pain and inflammation, especially in peripheral joints such as the toes or fingers.

"Gout" refers to a group of disorders or symptoms most often associated with the accumulation of uric acid due to an overproduction of uric acid or a reduced ability of the kidney to excrete uric acid. Gout is often characterized by the deposition of urate crystals (uric acid or salts thereof, e.g. monosodium urate) in the joints (gouty arthropathy) or soft tissue (tophi). "Gout" as used herein includes acute gout, chronic gout, moderate gout, refractory gout and severe gout.

"Gout-associated inflammation" refers to local or systemic inflammation due to immune responses to the deposition of urate crystals.

"Halofenate" refers to compounds of Formula (III) below, i.e. (4-chlorophenyl)-(3-trifluoromethylphenoxy)-acetic acid 2-acetylaminoethyl ester (also referred to as the 2-acetamidoethyl ester of 4-chlorophenyl-(3-trifluoromethylphenoxy)-acetic acid. The term halofenate and the corresponding chemical names include both the (+) and (-) enantiomer of compounds of Formula (III) as well as mixtures thereof, unless otherwise specified.

"Halofenic acid" and "CPTA" refer to the compounds of Formula (IV), i.e. 4-chlorophenyl-(3-trifluoromethylphenoxy)-acetic acid [also referred to as 2-(4-chlorophenyl)-2-(3-(trifluoromethyl)phenoxy)acetic acid] as well as its pharmaceutically acceptable salts. The term halofenic acid and the corresponding chemical names include both the (+) and (-) enantiomer of compounds of Formula (IV) as well as mixtures thereof, unless otherwise specified.

"Hyperuricemia" refers to an elevated serum uric acid level (see above).

"Impaired renal function" refers to a medical condition in which the kidneys fail to adequately filter toxins and waste products from the blood. Impaired renal function may take the form of acute kidney injury or chronic kidney disease (i.e. CKD1-5).

"Moderate gout" refers to gout present in a subject having at least two gout flares in the past 12 months.

"Pharmaceutically acceptable" refers to that which is useful in preparing a pharmaceutical composition that is generally safe, non-toxic, and neither biologically nor otherwise undesirable, and includes that which is acceptable for veterinary or human pharmaceutical use.

"Pharmaceutically acceptable salt" includes pharmaceutically acceptable acid addition salts and pharmaceutically acceptable base addition salts and includes both solvated and unsolvated forms. Representative non-limiting lists of pharmaceutically acceptable salts can be found in S.M. Berge et al., J. Pharma Sci., 66(1), 1-19 (1977), and Remington: The Science and Practice of Pharmacy, R. Hendrickson, ed., 21st edition, Lippincott, Williams & Wilkins, Philadelphia, PA, (2005), at p. 732, Table 38-5.

"Pharmaceutically acceptable acid addition salt" refers to salts formed with inorganic acids such as hydrochloric acid, hydrobromic acid, sulfuric acid, nitric acid, phosphoric acid and the like, and organic acids such as acetic acid, trifluoroacetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like.

"Pharmaceutically acceptable base addition salt" refers to salts prepared from the addition of an inorganic base or an organic base to the free acid. Salts derived from inorganic bases include, but are not limited to, the sodium, potassium, lithium, ammonium, calcium, magnesium, iron, zinc, copper, manganese, aluminum salts and the like. Salts derived from organic bases include, but are not limited to, salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-dimethylaminoethanol, 2-diethylaminoethanol, dicyclohexylamine, lysine, arginine, histidine, caffeine, procaine, hydrabamine, choline, betaine, ethylenediamine, glucosamine, methylglucamine, theobromine, purines, piperazine, piperidine, N-ethylpiperidine, polyamine resins and the like.

"Refractory gout" refers to gout in patients who are unresponsive or poorly responsive to one or more second urate-lowering agents, or have experienced or are at an increased risk of experiencing an adverse event therefrom. The terms "unresponsive" and "poorly responsive" in this context include (1) no or insignificant lowering of serum uric acid, (2) failure to reach a target serum uric acid level (e.g. as determined by a physician or other medical practitioner), and (3) the persistence of one or more gouty conditions or symptoms such as gout flares, gouty tophus, gouty arthritis, or other associated conditions regardless of any lowering of serum uric acid levels.

"Second urate-lowering agent" refers to a therapeutic agent that lowers serum uric acid levels that is not a first urate-lowering agent. Second urate-lowering agents include currently available agents (i.e. an agent approved by the FDA or other appropriate regulatory authority as of the filing date of this application) that lower serum uric acid, as well as compounds currently in development or under regulatory review. Examples of second urate-lowering agents are provided below. For clarity, this term implies no temporal aspect or relationship, e.g. to a first urate-lowering agent.

"Subject" and "patient" refer to animals such as mammals, including humans, other primates, domesticated animals (e.g. dogs, cats), farm animals (e.g. horses, cattle, goats, sheep, pigs), rats and mice.

"Severe gout" refers to gout present in a subject having tophaceous deposits in the joints, skin, or kidneys resulting in chronic arthritis, joint destruction, subcutaneous tophi, or kidney dysfunction, and, in some cases, with subsequent deformity and/or disability.

"Substantially free from" when used in reference to (-)-halofenate or (-)-halofenic acid (or a salt thereof) being substantially free from the corresponding (+) enantiomer (i.e. (+)-halofenate, (+)-halofenic acid, or a salt thereof) refers to a composition containing a high proportion of a compound's (-) enantiomer in relation to the (+) enantiomer . In one embodiment, the term means that by weight, the compound included in the composition is at least 85% (-) enantiomer and at most 15% (+) enantiomer. In one embodiment, the term means that by weight, the compound included in the composition is at least 90% (-)enantiomer and at most 10% (+) enantiomer. In other embodiments, the term means that by weight, the compound included in the composition is at least 91% (-)enantiomer and at most 9% (+) enantiomer, at least 92% (-) enantiomer and at most 8% (+) enantiomer, at least 93% (-) enantiomer and at most 7% (+) enantiomer, at least 94% (-) enantiomer and at most 6% (+) enantiomer, at least 95% (-) enantiomer and at most 5% (+) enantiomer, at least 96% (-) enantiomer and at most 4% (+) enantiomer, at least 97% (-) enantiomer and at most 3% (+) enantiomer, at least 98% (-) enantiomer and at most 2% (+) enantiomer, or at least 99% (-) enantiomer or greater than 99% (-) enantiomer. Other percentages of the (-) and (+) enantiomers may also be provided. These percentages are based upon the amount of the enantiomer relative to the total amount of both enantiomers of the compound in the composition.

"Therapeutically effective dose", "therapeutically effective amount", or, interchangeably, "pharmacologically acceptable dose" and "pharmacologically acceptable amount" mean that a sufficient amount of a therapeutic agent, therapeutic agents, or metabolites thereof will be present in order to achieve a desired result, e.g., lowering uric acid levels to a target goal or treating gout in its various forms or treating conditions associated with hyperuricemia.

"Treatment" and "treating" of a disease, disorder, condition or symptom refer to (1) preventing or reducing the risk of developing the disease, disorder or condition, i.e., causing the clinical symptoms of the disease, disorder or condition not to develop in a subject who may be exposed to or predisposed to the disease, disorder or condition but who does not yet experience or display symptoms of the disease, disorder or condition (i.e. prophylaxis); (2) inhibiting the disease, disorder or condition, i.e., arresting or reducing the development of the disease, disorder or condition or its clinical symptoms; and (3) relieving the disease, disorder or condition, i.e., causing regression, reversal, or amelioration of the disease, disorder or condition or reducing the number, duration, frequency or intensity one or more of its clinical symptoms (e.g. a gout flare). The term "management" may be used synonymously.

"Urate" refers to uric acid (7,9-dihydro-1H-purine-2,6,8(3H)-trione) and ions and salts thereof.

This application describes methods of treating a gout flare comprising administering to the subject a compound of Formula (I) 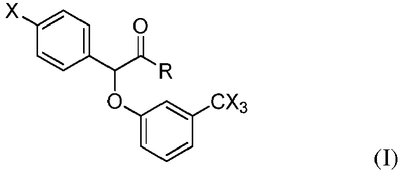 wherein R is selected from the group consisting of a hydroxy, lower aralkoxy, di-lower alkylamino-lower alkoxy, lower alkanamido-lower alkoxy, benzamido-lower alkoxy, ureido-lower alkoxy, N'-lower alkyl-ureido-lower alkoxy, carbamoyl-lower alkoxy, halophenoxy substituted lower alkoxy, carbamoyl substituted phenoxy, carbonyl-lower alkylamino, N,N-di-lower alkylamino-lower alkylamino, halo substituted lower alkylamino, hydroxy substituted lower alkylamino, lower alkanolyloxy substituted lower alkylamino, ureido, and lower alkoxycarbonylamino; and each X is independently a halogen, or a pharmaceutically acceptable salt thereof.

The compound may be a compound of Formula (II) 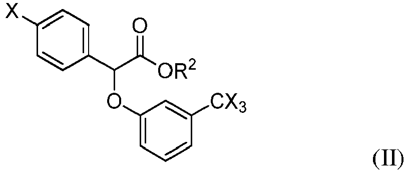 wherein R² is selected from the group consisting of phenyl-lower alkyl, lower alkanamido-lower alkyl, and benzamido-lower alkyl; and each X is independently a halogen, or a pharmaceutically acceptable salt thereof.

In some aspects, the compound is a compound of Formula (III), also referred to as halofenate 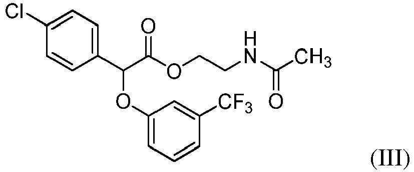 or a pharmaceutically acceptable salt thereof.

In other aspects, the compound is a compound of Formula (IV), also referred to as halofenic acid 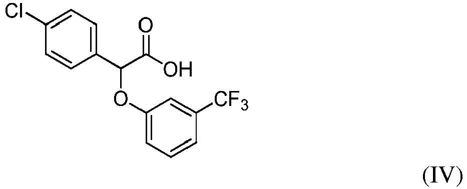 or a pharmaceutically acceptable salt thereof.

It should be noted that any carbon atom with unsatisfied valences in the formulae and examples herein is assumed to have the hydrogen atom to satisfy the valences.

In certain embodiments the compound is a compound that generates the compound of Formula (IV) or a pharmaceutically acceptable salt thereof via a chemical reaction after being administered, as discussed in more detail below.

The compound may be the (-) enantiomer of a compound of Formulae (I), (II), (III), or (IV). In certain embodiments, the compound is (-)-halofenate (i.e. (-)-(*R*)-(4-chloro-phenyl)-(3-trifluoromethyl-phenoxy)-acetic acid 2-acetylamino-ethyl ester, also referred to as arhalofenate). In other embodiments, the compound is (-)-halofenic acid (i.e. (-)-4-chlorophenyl-(3-trifluoromethylphenoxy) acetic acid) or a pharmaceutically acceptable salt thereof. The (-)- halofenate, (-)-halofenic acid, or pharmaceutically acceptable salt thereof is substantially free from the corresponding (+) enantiomer.

The enantiomers (stereoisomers) of compounds of Formulae (I), (II), (III), or (IV) and pharmaceutically acceptable salt thereof can be prepared by using reactants or reagents or catalysts in their single enantiomeric form in the process wherever possible or by resolving the mixture of stereoisomers by conventional methods including use of microbial resolution, resolving the diastereomeric salts formed with chiral acids or chiral bases and chromatography using chiral supports. *See,* also U.S. Patent No. 7,199,259 (Daugs), U.S. Patent Nos. 6,646,004; 6,624,194; 6,613,802; and 6,262,118 (each to Luskey et al.), U.S. Patent No. 7,714,131 (Zhu et al.), U.S. Patent No. 7,432,394 (Cheng et al.) and U.S. Publication No. 2010/0093854 (Broggini et al.)

The chemical synthesis of racemic mixtures of (3-trihalomethylphenoxy) (4-halophenyl) acetic acid derivatives can also be performed by the methods described in U.S. Patent No. 3,517,050. The individual enantiomers can be obtained by resolution of the racemic mixture of enantiomers using conventional means known to and used by those of skill in the art. *See, e.g.,* J. Jaques et al., in ENANTIOMERS, RACEMATES, AND RESOLUTIONS, John Wiley and Sons, New York (1981). Other standard methods of resolution known to those skilled in the art, including but not limited to, simple crystallization and chromatographic resolution, can also be used *(see, e.g.,* STEREOCHEMISTRY OF CARBON COMPOUNDS (1962) E. L. Eliel, McGraw Hill; J. Lochmuller, Chromatography 113, 283-302 (1975)). Additionally, halofenate, halofenic acid, or a pharmaceutically acceptable salt thereof, *i.e.,* the optically pure isomers, can be prepared from the racemic mixture by enzymatic biocatalytic resolution. Enzymatic biocatalytic resolution has been generally described previously (*see, e.g.,* U.S. Patent Nos. 5,057,427 and 5,077,217). Other generic methods of obtaining enantiomers include stereospecific synthesis (*see, e.g.,* A. J. Li et al., Pharm. Sci. 86, 1073-1077 (1997)).

While not wanting to be bound to any particular theory, it is thought that several properties of the compounds described herein account for their successful use in the treatment (including reducing the number, duration, frequency or intensity) of gout flares, for example during the initiation of and maintenance use of these compounds for uric acid lowering: (1) their pharmacokinetic profile and (2) their anti-inflammatory properties including inhibitory effect on interleukin-lbeta (IL-1b) and reduction of high sensitivity C-reactive protein, and (3) their ability to block uric acid entry into a cell.

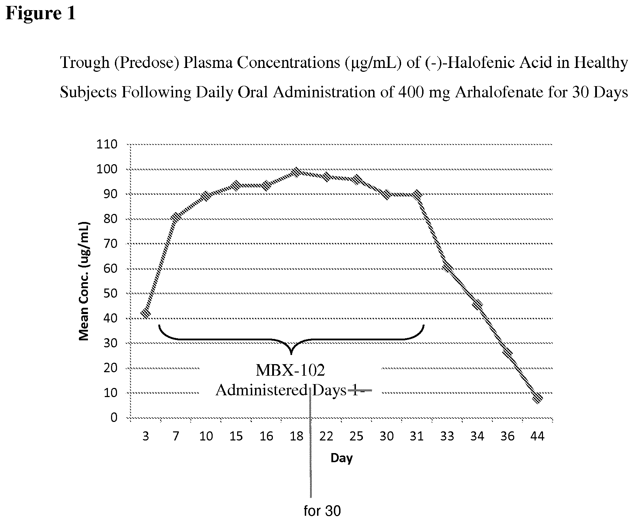

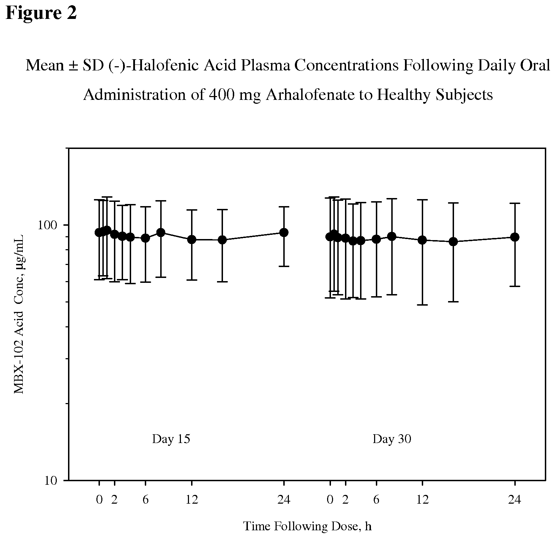

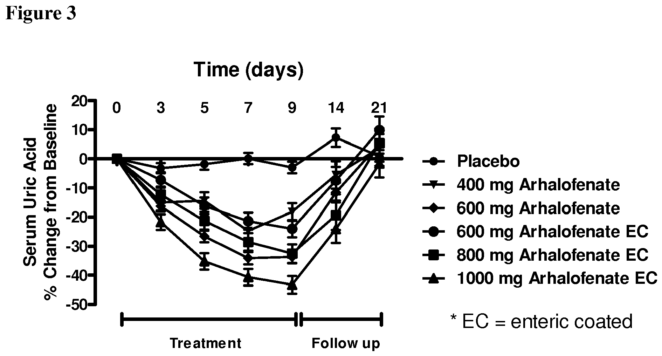

FIGS. 1-2 show the pharmacokinetic profile of (-)-halofenic acid. FIG. 1 shows the mean trough plasma concentration values of (-)-halofenic acid during and following a 30-day dosing schedule of daily oral administration of 400 mg of arhalofenate. FIG. 2 shows the mean and standard deviation (SD) plasma concentration values of (-)-halofenic acid at day 15 and day 30 following daily oral administration of 400 mg of arhalofenate. These figures demonstrate a long half-life with sustained drug levels present for several days after the final dose, and a relatively constant intraday plasma concentration. The plasma concentration of (-)-halofenic acid is expected to correlate to the plasma concentration of uric acid. Accordingly, the long half-life and low intraday peak-to-trough ratio are expected to result in correspondingly gradual changes in serum uric acid during the initiation of and maintenance use of therapy. FIG. 3 demonstrates the reduction in serum uric acid over time with several doses of arhalofenate, and supports this theory. It is thought that large or rapid changes in serum uric acid (resulting from, for example, certain second urate-lowering agents, e.g. allopurinol, febuxostat, and others) can trigger gout flares or result in longer, more frequent, or more intense flares, for example during and for the several weeks and months after initiation of such agents, or with non-adherance to daily use of such agents. Therefore, the pharmacokinetic profile of (-)-halofenic acid should contribute to the successful use of compounds of Formulae (I), (II), (III), and (IV) and pharmaceutically acceptable salts thereof in the prevention of gout flares (for example, during certain durations such as the first several weeks to month after initiation of administration), compared to other urate lowering therapies.

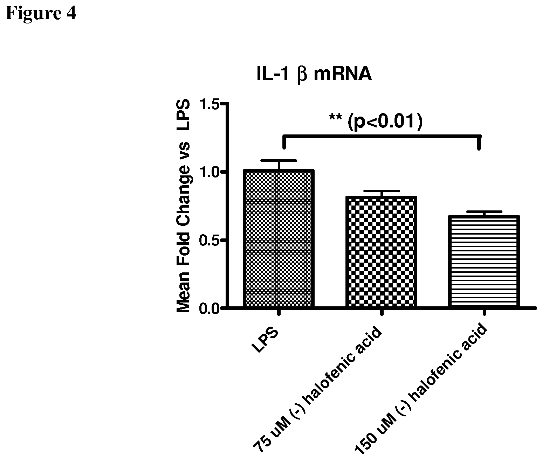

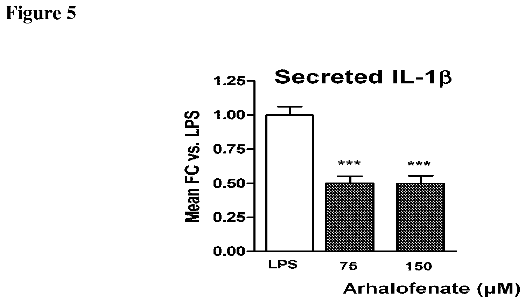

The compounds of Formulae (I), (II), (III), and (IV) and pharmaceutically acceptable salts thereof also have an inhibitory effect on and block key pathways of inflammation. Inflammasomes are molecular platforms activated upon cellular infection or stress that trigger the maturation of proinflammatory cytokines such as interleukin-lbeta (IL-1β) to engage innate immune defenses. Thus, inhibitors of IL-1β may have a role in gout therapy. *See, e.g.,* A. So. and N. Busso, Ann. Rheum. Dis., 68(10) (2009) and references cited therein. FIG. 4 shows the effect of (-)-halofenic acid to lower the messenger RNA encoding the pro-inflammatory cytokine IL-1β in lipopolysaccharide (LPS) stimulated primary mouse macrophages. It is hypothesized that (-)-halofenic acid exerts its anti-inflammatory effects by binding to the transcription factor PPAR-γ and in this liganded form directly interacting with and stabilizing the transcriptional machinery located on the promoter for IL-1β. In this way the activation of the Il-1β promoter in response to LPS treatment is prevented by (-)-halofenic acid. FIG. 5 shows the effect of (-)-halofenic acid on lowering the secretion of the pro-inflammatory cytokine IL-1β from lipopolysaccharide stimulated primary mouse macrophages.

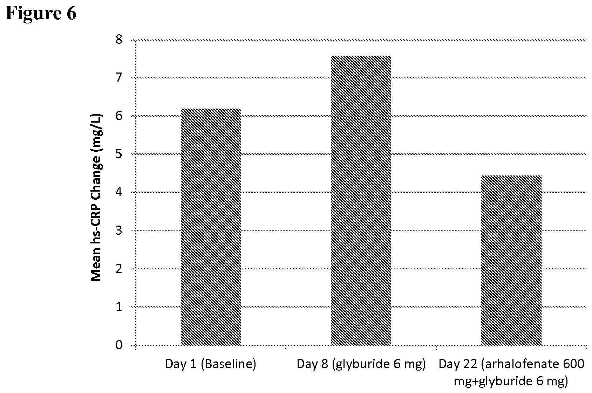

High-sensitivity C-reactive protein (hs-CRP) is another marker of inflammation. FIGS. 6-7 show that administration of (-)-halofenate in human subjects lowered hs-CRP, further supporting the effectiveness of compounds of Formulae (I), (II), (III), or (IV) or pharmaceutically acceptable salts or prodrugs thereof to treat gout flares.

An additional mechanism contributing to the successful treatment of gout flares is the blocking of entry of uric acid into a cell. Uric acid transport into cells is mediated by a number of uric acid transporters including URAT1, GLUT9 (SLC2A9), OAT4 and OAT10. For example, in adipocytes uric acid increases the expression of pro-inflammatory cytokines such as MCP-1 (Baldwin et al., Diabetes 60 1258-1269 (2011)). It is hypothesized that (-)-halofenic acid is a URAT1 inhibitor and as such, administration of compounds of Formulae (I), (II), (III), and (IV) and pharmaceutically acceptable salts thereof would be expected to decrease uric-acid induced inflammatory responses in cells, including, for example, adipocytes, macrophages and endothelial cells.

Methods described herein include reducing the number, duration, frequency or intensity of one or more gout flares, the methods comprising administering to a subject in need thereof a compound of any of Formulae (I), (II), (III), or (IV) or a pharmaceutically acceptable salt thereof. In some embodiments the compound is (-)-halofenate, (-)-halofenic acid or a pharmaceutically acceptable salt thereof. In some embodiments, the number, duration, frequency or intensity of gout flares experienced by the subject is reduced relative to that experienced by the subject before such administration is initiated. In other embodiments, the number, duration, frequency or intensity of gout flares experienced by the subject is reduced relative to the number, duration, frequency or intensity of gout flares experienced by the subject when the subject has previously undergone urate-lowering therapy with a second urate-lowering agent. In certain methods of reducing the number, duration, frequency or intensity of gout flares experienced by the subject, the amount or duration of administration of any additional therapeutic agent (e.g. a flare prophylaxis agent such as a non-steroidal anti-inflammatory drug (NSAID) or colchicine) is reduced, and in other such methods no such additional therapeutic agent (e.g. NSAID or colchicine) is administered.

The second urate-lowering agent may be any agent that lowers serum uric acid levels that is not a first urate-lowering agent (i.e. not a compound of any of Formulae (I), (II), (III), or (IV) or a pharmaceutically acceptable salt thereof). These second urate-lowering agents include inhibitors of uric acid production (e.g. xanthine oxidase inhibitors and purine nucleoside phosphorylase inhibitors), uricosuric agents, and uricases. Xanthine oxidase inhibitors include, but are not limited to: allopurinol, febuxostat, oxypurinol, tisopurine, an inositol and propolis. In some embodiments, the xanthine oxidase inhibitor is allopurinol, febuxostat, oxypurinol, tisopurine, inositol, phytic acid, myo-inositiol, kaempferol, myricetin, and quercetin. Allopurinol (1,5-dihydro-4H-pyrazolo [3,4-d]pyrimidin-4-one), a xanthine oxidase inhibitor, is the current first line standard of care for lowering urate levels. Another xanthine oxidase inhibitor, febuxostat (2-(3-cyano-4-isobutoxyphenyl)-4-methyl-1,3-thiazole-5-carboxylic acid), was approved for treatment of gout in February 2009. Purine nucleoside phosphorylase (PNP) inhibitors represent a relatively new approach to lowering serum uric acid levels in patient with hyperuricemia, gout, and related conditions. In some embodiments, the PNP inhibitor is forodesine (BCX-1777) (BioCryst Pharmaceuticals, Inc.). In other embodiments, the PNP inhibitor is BCX-4208 (7-(((3R,4R)-3-hydroxy-4-(hydroxymethyl)pyrrolidin-1-yl)methyl)-3H-pyrrolo[3,2-d]pyrimidin-4(5H)-one) (BioCryst Pharmaceuticals, Inc.). BCX4208 monotherapy administered at 40, 80, 120, 160 and 240 mg/day has been shown to rapidly and significantly reduced serum uric acid in gout patients. Uricosuric agents enhance renal excretion of uric acid and generally act by lowering the absorption of uric acid from the kidney proximal tubule back to the blood, e.g., by inhibiting urate transporters, e.g, SLC22A12. Uricosuric agents include, but are not limited to, probenecid, 2-((5-bromo-4-(4-cyclopropylnaphthalen-1-yl)-4H-1,2,4-triazol-3-yl)thio)acetic acid (RDEA594, lesinurad), potassium 4-(2-((5-bromo-4-(4-cyclopropylnaphthalen-1-yl)-4H-1,2,4-triazol-3-yl)thio)acetamido)-3-chlorobenzoate (RDEA806), RDEA684, benzbromarone, sulfinpyrazone, amlodipine, atorvastatin, fenofibrate, guaifenesin, losartan, adrenocorticotropic hormone, and cortisone. Probenecid is the most commonly used uricosuric agent in the U.S. and may be given in combination with allopurinol to some gout patients. Benzbromarone and sulfinpyrazone are also used as first line uricosuric agents. Guaifenesin, losartan, atorvastatin, amlodipine, adrenocorticotropic hormone (ACTH or corticotropin), fenofibrate and cortisone also have uricosuric effects. Uricase or urate oxidase enzymes are found in many mammals but not humans. They can lower uric acid levels by converting uric acid into allantoin, a benign end metabolite which is easily excreted in the urine. Uricase enzymes include, but are not limited to, rasburicase or a pegylated uricase enzyme (PEG-uricase). In some embodiments, the pegylated uricase enzyme is Krystexxa® (PURICASE®; pegloticase) (Savient Pharmaceuticals, Inc.) which is approved in the U.S. for the treatment of chronic gout in adult patients refractory to conventional therapy.

In some embodiments, the number of gout flares experienced by the subject is reduced relative to the number, duration, frequency or intensity of gout flares experienced by the subject when the subject has previously undergone urate-lowering therapy with a second urate-lowering agent, wherein the second urate-lowering agent is allopurinol, febuxostat, lesinurad or BCX4208.

Certain methods provide for the treatment or management of hyperuricemia in a subject with gout and reducing the number, duration, frequency or intensity of gout flares experienced by the subject. These methods comprise administering to a subject in need thereof a compound of any of Formulae (I), (II), (III), or (IV) or a pharmaceutically acceptable salt thereof. In some embodiments the compound is (-)-halofenate, (-)-halofenic acid or a pharmaceutically acceptable salt thereof.

In various embodiments, the methods described herein lower serum uric acid levels in a subject by about 5%, about 10%, about 15%, about 20%, about 25%, about 30%, about 35%, about 40%, about 45%, about 50%, about 55%, about 60%, about 65%, about 70%, about 75%, about 80%, about 85%, about 90% or more, as compared to serum uric acid levels in the subject prior to administering the methods described herein. In various embodiments, serum uric acid levels are decreased about 5% to about 50%, decreased by about 25% to about 75%, or decreased by about 50% to about 99%. Methods to determine serum uric acid levels are well known in the art and are often measured as part of a standard chemistry panel of blood serum samples.

In some embodiments, the methods of the present disclosure lower serum uric acid levels in a subject to about 7 mg/dL or less, to about 6.5 mg/dL or less, to about 6 mg/dL or less, to about 5 mg/dL or less, to about 4 mg/dL or less, or to about 3 mg/dL or less as compared to serum uric acid levels in the subject prior to administering the methods or compositions described herein. In some embodiments, the methods of the present disclosure lower serum uric acid levels in a subject by 0.1, 0.2, 0.3, 0.4, 0.5, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5 or 10.0 mg/dL, or greater, as compared to serum uric acid levels in the subject prior to administering the methods or compositions described herein. In further embodiments, the methods described herein lower serum uric acid levels by between 0.1 and 10.0 mg/dL, between 0.5 and 6.0 mg/dL, between 1.0 and 4.0 mg/dL or between 1.5 and 2.5 mg/dL. The appropriate serum uric acid level may vary depending on the subject, and may vary for a given subject over time, depending upon the subject's overall medical condition. Similarly, the appropriate serum uric acid level for one group of subjects sharing a common medical condition may be different from that which is appropriate for a different group of subjects sharing a different medical condition. Thus, it may be advisable to reduce the serum uric acid level of a given group of subjects to, for example, below about 5 mg/dL, and to reduce the serum uric acid level of a different group of subjects to, for example, below about 4 mg/dL. In certain embodiments, the methods of the present disclosure decrease a serum uric acid level in the subject by an amount sufficient to result in the disappearance, reduction, amelioration, or the prevention of the onset, of one or more conditions associated with elevated serum uric acid over a certain timeframe, for example about a week, about a month, about six months, about one year, about two years, or for a longer duration. For example, a method can decrease the serum uric acid level in a subject by an amount sufficient to result in the disappearance or reduction of tophi over about one week, about one month, about six months, about one year, about two years, or longer, e.g. indefinitely, e.g. for the remainder of the lifetime of the subject.

In further embodiments, the methods of the present disclosure comprise administering a pharmaceutical composition comprising a compound of Formulae (I), (II), (III) or (IV) or a pharmaceutically acceptable salt thereof to a subject whose serum uric acid level is at least about 4 mg/dL, at least about 5 mg/dL, at least about 6 mg/dL, at least about 6.8 mg/dL, at least about 7 mg/dL, at least about 8 mg/dL, at least about 9 mg/dL, at least about 10 mg/dL, or at least about 11 mg/dL. Again, the amount of decrease of serum uric acid level that is appropriate may vary depending on the subject, depending upon the subject's overall medical condition. Similarly, the amount of decrease of serum uric acid level that is appropriate for one group of subjects sharing a common medical condition may be different from that which is appropriate for a different group of subjects sharing a different medical condition.

The methods described herein (as well as the underlying physiological mechanisms related to them) may be accomplished by the administration of a compound that generates the compound of Formula (IV) or a salt thereof via a chemical reaction after being administered. Such compounds include prodrugs of the compound of Formula (IV). Prodrugs of a compound are prepared by modifying functional groups present in the compound in such a way that the modifications may be cleaved *in vivo* to release the parent compound, or an active metabolite. For example, prodrugs include compounds wherein a hydroxy, amino, or sulfhydryl group in a compound is bonded to any group that may be cleaved in vivo to regenerate the free hydroxyl, amino, or sulfhydryl group, respectively. Certain prodrugs may increase the bioavailability of the compounds of the embodiments when such compounds are administered to a subject (e.g., by allowing an orally administered compound to be more readily absorbed into the blood) or which enhance delivery of the parent compound to an organ or tissue (e.g., adipose tissue, kidneys, liver, muscle, or joints) relative to the parent species. More particularly, prodrugs of the compound of Formula (IV) include esters, amides, and carbamates (e.g., N, N-dimethylaminocarbonyl) of the hydroxy functional group of the compound of Formula (IV). The compounds of Formulae (I), (II), and (III) are non-limiting examples of prodrugs of the compound of Formula (IV). Further examples of prodrugs can be found in J. Rautio et al. Prodrugs: design and clinical applications, Nat. Rev. Drug Discov., 7, 255-270 (2008); Edward B. Roche, ed., Bioreversible Carriers in Drug Design, American Pharmaceutical Association and Pergamon Press, (1987); and T. Higuchi and V. Stella, Pro-drugs as Novel Delivery Systems, Vol. 14 of the A.C.S. Symposium Series (1975).

The compounds of Formulae (I), (II), (III) and (IV) and pharmaceutically acceptable salts thereof are contemplated to exhibit therapeutic activity when administered in an amount which can depend on the particular case. The variation in amount can depend, for example, on the subject being treated and the active ingredients chosen. A broad range of doses can be applicable. Dosage regimes may be adjusted to provide the optimum therapeutic response. For example, several divided doses may be administered daily, weekly, monthly or other at suitable time intervals or the dose may be proportionally reduced as indicated by the exigencies of the situation. Such dosages are optionally altered depending on a number of variables, not limited to the activity of the one or more active ingredients used, the disease or condition to be treated, the mode of administration, the requirements of the individual subject, the severity of the disease or condition being treated, and the judgment of the practitioner.

Depending on factors such as the diagnosis, symptoms, and therapeutic goals of a particular subject, a wide range of dosages of the compound of Formulae (I), (II), (III), or (IV) can be contemplated. In various embodiments, the compound may be administered from about 10 mg to about 1000 mg per day. For example, halofenate, halofenic acid, or a pharmaceutically acceptable salt thereof may be administered at about 50 mg/day, about 100 mg/day, about 200 mg/day, about 300 mg/day, about 400 mg/day, about 500 mg/day, about 600 mg/day, about 700 mg/day, about 800 mg/day, about 900 mg/day, or about 1000 mg/day.

Dose titration or dose escalation protocols may be employed to determine the proper or optimal dose to administer to a subject. For example, dose titration or escalation studies may select for doses that improve efficacy or tolerability. Dose titration or escalation allows for the gradual adjusting of the dose administered until the desired effect is achieved. Dose titration gradually decreased the dosage administered while dose escalation gradually increases the dose administered. Methods of dose titration and escalation are well known in the art. As a non-limiting example, a subject may be administered 200 mg/day halofenate, halofenic acid, or a pharmaceutically acceptable salt thereof every day and measured for serum uric acid levels on a daily basis. The dosage may be increased or decreased, for example, on a weekly basis. The subject may be monitored for a period of, for example, 2 to 12 weeks to find the desired dose.

Compounds of Formula (I), (II), (III) or (IV) can be incorporated into a variety of formulations and medicaments for therapeutic administration. More particularly, these compounds can be formulated into pharmaceutical compositions or formulations by combination with appropriate, pharmaceutically acceptable carriers or diluents, and can be formulated into preparations in solid, semi-solid, liquid or gaseous forms, such as tablets, capsules, pills, powders, granules, dragees, gels, slurries, ointments, solutions, suppositories, injections, inhalants and aerosols. As such, administration of the compounds can be achieved in various ways, including oral, buccal, rectal, parenteral, intraperitoneal, intradermal, transdermal, or intratracheal administration. Moreover, the compound can be administered in a local rather than systemic manner, in a depot or sustained release formulation. In addition, the compounds can be administered in a liposome.

Compounds of Formula (I), (II), (III) or (IV) or a pharmaceutically acceptable salt thereof can also be formulated with common excipients, diluents or carriers and compressed into tablets, or formulated as elixirs or solutions for convenient oral administration, or administered by the intramuscular or intravenous routes. The compounds can be administered transdermally, and can be formulated as sustained release dosage forms and the like. The above methods may further comprise the administration of a second urate-lowering agent selected from the group consisting of a xanthine oxidase inhibitor, an inhibitor of uric acid production, a uricosuric agent and a uricase. The method may comprise administering a pharmaceutical composition comprising a first urate-lowering agent and a second therapeutic agent, as described herein, to a subject whose serum uric acid level is at least about 4 mg/dL, at least about 5 mg/dL, at least about 6 mg/dL, at least about 6.8 mg/dL, at least about 7 mg/dL, at least about 8 mg/dL, at least about 9 mg/dL, at least about 10 mg/dL, or at least about 11 mg/dL. The amount of decrease of serum uric acid level that is appropriate may vary depending on the subject, depending upon the subject's overall medical condition. Similarly, the amount of decrease of serum uric acid level that is appropriate for one group of subjects sharing a common medical condition may be different from that which is appropriate for a different group of subjects sharing a different medical condition. The the application provides combination therapy and methods of concomitant administration of a first and second urate-lowering agent (wherein these first and second urate-lowering agents are described herein). Combination therapy and concomitant administration refer to the administration of the two agents (i.e., a first agent and a second urate-lowering agent, as described herein) in any manner in which the pharmacological effects of both are manifested in the subject at the same time. Thus, such administration does not require that a single pharmaceutical composition, the same type of formulation, the same dosage form, or even the same route of administration be used for administration of both the first and second urate-lowering agents, or that the two agents be administered at the same time. Such administration may be accomplished most conveniently by the same dosage form and the same route of administration, at substantially the same time. For example, a first urate-lowering agent, e.g. halofenate, halofenic acid, or a pharmaceutically acceptable salt thereof, and a second urate-lowering agent, e.g. xanthine oxidase inhibitor (e.g., allopurinol or febuxostat), can be administered to the human subject together in a single oral dosage composition, such as a tablet or capsule, or each agent can be administered in separate oral dosage formulations. One advantage with separate formulations is an added flexibility in dosing, i.e. the dosage of the first and second urate-lowering agents can be changed independently, quickly, and easily. Where separate dosage formulations are used, the first and second urate-lowering agents can be administered at essentially the same time (i.e., simultaneously or concurrently), or at separately staggered times (i.e., sequentially). In another embodiment, the second urate-lowering agent is a xanthine oxidase inhibitor, preferably selected from the group consisting of allopurinol, febuxostat, oxypurinol, tisopurine, inositol, phytic acid, myo-inositiol, kaempferol, myricetin, and quercetin, especially allopurinol or febuxostat. In yet another embodiment, the second urate-lowering agent is allopurinol and is administered at from about 50 mg to about 800 mg per day. In another embodiment, the first urate-lowering agent is (-)-halofenate and is administered at from about 100 mg to about 600 mg per day, and the second urate-lowering agent is febuxostat and is administered at from about 40 mg to about 120 mg per day. In another embodiment, the second urate-lowering agent is a uricosuric agent, preferably selected from the group consisting of probenecid, 2-((5-bromo-4-(4-cyclopropylnaphthalen-1-yl)-4H-1,2,4-triazol-3-yl)thio)acetic acid, potassium 4-(2-((5-bromo-4-(4-cyclopropylnaphthalen-1-yl)-4H-1,2,4-triazol-3-yl)thio)acetamido)-3-chlorobenzoate, RDEA684, benzbromarone, sulfinpyrazone, amlodipine, atorvastatin, fenofibrate, guaifenesin, losartan, adrenocorticotropic hormone and cortisone, especially probenecid.

In various embodiments, compounds of Formula (I), (II), (III) or (IV) or a pharmaceutically acceptable salt thereof can be administered over a broad frequency range. For example, in various embodiments the compounds may be administered once daily (QD), twice daily (BID), three times daily (TID) or four times per day (QID). In one embodiment, the compound is administered once daily (QD). In another embodiment, the compound is administered twice daily (BID). In other embodiments, administration of the compound can be skipped without having deleterious effect, that is, the compound can be administered over (i.e. before and after) a "drug holiday" where the drug holiday is the period of the skipped dose. For example, in a daily dosing regimen, the compound can be administered over a drug holiday of one day, (i.e. administered on day N and day N+2 but not on day N+1, where day N is any arbitrary day) without the subject experiencing any substantially or materially adverse effect from the skipped administration. In certain embodiments the drug holiday can be two days. In other embodiments the drug holiday can be more than two days.

In various embodiments, compounds of Formula (I), (II), (III) or (IV) or a pharmaceutically acceptable salt thereof can be administered over a broad duration. For example, in various embodiments the compounds may be administered for about four weeks or longer, about one month or longer, about three months or longer, for about six months or longer, for about one year or longer, for about two years or longer, for about five years or longer, or for about ten years or longer. In some embodiments the administration may be indefinite, e.g. for the remainder of the lifetime of the subject.

The pharmacokinetic profile of (-)-halofenic acid can be modulated by the dose, frequency, and duration of administration of the compound or a prodrug thereof. One measure of the pharmacokinetic profile is the peak-to-trough ratio, defined as the highest blood plasma concentration divided by the lowest blood plasma concentration of a compound or agent within a certain time interval (e.g. within the interval corresponding to the frequency of administration). For example, certain methods include providing to a subject an intraday peak-to-trough ratio of (-)-halofenic acid of about 2.0 or less, comprising administering to the subject a compound of Formulae (I), (II), (III) and (IV) or pharmaceutically acceptable salts thereof at a dose of about 100 to about 1000 mg per day. In various embodiments, the intraday peak-to-trough ratio is about 1.7 or less, about 1.5 or less, about 1.4 or less, or about 1.3 or less. In embodiments, the intraday peak-to-trough ratio is provided after administering the compound daily for at least about 10 days, e.g. at least about 12 days. The pharmacokinetic profile can also depend on the route of administration, as well as by the compound and formulation administered to the subject. For example, one method includes providing to a subject an intraday peak-to-trough ratio of (-)-halofenic acid of about 2.0 or less, comprising administering to the subject arhalofenate (i.e. (-)-halofenate) by mouth in an oral formulation (e.g. a tablet, capsule, pill, etc. as described above) at a dose of 100 to 1000 mg per day.

Certain methods described herein may be accomplished by administering a compound of Formulae (I), (II), (III) and (IV) or pharmaceutically acceptable salts thereof at a certain dose, frequency, and duration of administration, as provided herein. For example, certain methods provide for the treatment of hyperuricemia in a subject with gout comprising administering to a subject in need thereof a compound of Formula (I), (II), (III) and (IV) or pharmaceutically acceptable salts thereof wherein the dose, frequency, and duration of administration are effective to reduce the number, duration, frequency, or intensity of gout flares experienced by the subject during the duration. In some embodiments, the compound is arhalofenate. In some embodiments the dose is about 100 mg to about 1000 mg. In some embodiments the frequency is daily. In some embodiments the duration is about four weeks or longer. In other embodiments the duration is about one month or longer, about three months or longer, about six months or longer, about one year or longer, for about two years or longer, for about five years or longer, or for about ten years or longer. In some embodiments the administration may be indefinite, e.g. for the remainder of the lifetime of the subject. In some embodiments the administration daily and over a drug holiday of one day. In some embodiments further comprise administering to the subject arhalofenate by mouth in an oral formulation. Particular embodiments covering compositions, formulations and their method of uses are disclosed in a PCT Patent Application entitled "Methods for Treating Hyperuricemia in Patents with Gout Using Halofenate or Halofenic Acid and a Second Urate-Lowering Agent" filed concurrently with the present application. The embodiments of this application are characterized by the specification and by the features of the Claims of this application as filed, and of corresponding pharmaceutical compositions, methods and uses of these compounds.

### Methods

Methods used in relation to FIGS. 1-2 showing the pharmacokinetic profile of (-)-halofenic acid were as follows:

Plasma proteins in human plasma samples containing of (-)-halofenic acid, an internal standard (I.S.) and heparin as the anticoagulant were precipitated with acetonitrile. The samples were vortex mixed, centrifuged and an aliquot was analyzed by reversed phase high performance liquid chromatography using a Phenomenex Polar RP column maintained at 45 °C. The mobile phase was nebulized using heated nitrogen in a Z-spray source/interface and the ionized compounds were detected using a tandem quadrupole mass spectrometer.

The plasma concentrations were rounded to the nearest one-tenth µg/mL before the calculations. Plasma samples with concentrations below the quantifiable limit of 1.0 µg/mL (BQL) were assigned values of zero.

Methods used in relation to FIG. 3 showing reduction in serum uric acid in subjects over time following once daily dosing with arhalofenate were as follows.

A single centre, Phase 1, placebo- and positive-controlled, double-blind, randomized, dose escalation study (MAD study, MBX102-2DM01011b) was conducted to evaluate the multiple-dose pharmacokinetics (PK) of (-)-halofenate administered as a daily dose orally for 10 days, at the protocol-specified doses in healthy adult subjects. A total of 119 subjects completed study treatment according to protocol: 6 subjects received MBX-102 100 mg/day for 10 days; 6 subjects received MBX-102 200 mg/day for 10 days; 9 subjects received MBX-102 400 mg/day for 10 days; 20 subjects received MBX-102 600 mg/day for 10 days; 10 subjects received MBX-102 600 mg Enteric Coated (EC) /day for 10 days; 9 subjects received MBX-102 800 mg EC/day for 10 days; 10 subjects received MBX-102 1000 mg EC/day for 10 days; 24 subjects received placebo treatment daily for 10 days; and 25 subjects received naproxen 500 mg b.i.d. monotherapy for 7 days. In this study, serum uric acid was measured at screening and on Days 1, 3, 5, 7, 9, 14, and 21.

**Table 1**

| **Treatment group** | **N** | **Mean uric acid at baseline (mg/dL)** | **Mean uric acid at Day 9 (mg/dL)** | **Mean change at Day 9 (mg/dL)** | **Mean % change** |
|---|---|---|---|---|---|
| Placebo | 23 | 4.70 | 4.66 | -0.04 | -1 |
| 400 mg | 10 | 4.91 | 3.89 | -1.02 | -21 |
| 600 mg | 20 | 5.04 | 3.40 | -1.65 | -33 |
| 600 mg EC | 10 | 5.61 | 3.92 | -1.69 | -30 |
| 800 mg EC | 9 | 5.60 | 3.83 | -1.77 | -32 |
| 1000 mg EC | 10 | 5.48 | 3.04 | -2.44 | -45 |

As shown in FIG. 3 and Table 1, data from the MBX102-2DM01011b study demonstrates that the treatment with (-)-halofenate resulted in gradual reduction in serum uric acid over a period of time at all dose levels tested, and in a dose dependent manner.

Methods used in relation to FIGS. 4-5 showing effects of (-)-halofenic acid IL-1β were as follows:

Eight week-old male C57BL/6J mice were injected intra-peritoneally with 2.5 mL of 3% thioglycollate. Three days after injection, mice were sacrificed by cervical dislocation. Macrophages were harvested immediately by injecting 5 mL of PBS into the intra-peritoneal cavity, surgically exposing the intra-peritoneal cavity and aspirating the intra-peritoneal fluid with a 1 mL syringe. Macrophages from multiple animals were pooled and centrifuged at 1500 rpm, 10 min at 4 °C. Contaminating red blood cells were removed from the pellet by lysis with 10 mL RBC lysis buffer for 5min at room temperature, followed by centrifugation at 1500 rpm, 10 min at 4 °C. The cells were washed once and re-suspended in RPMI 1640, 10% FBS/0.1% Pen/Strep and plated into multi-well plates (1,000,000 cells/well for 24 well plates and 100,000 cells/well for 96 well plates). The cells were cultured for 30 hours, re-fed with fresh RPMI 1640, 0.5% FBS and incubated overnight. The cells were treated for 1 hour with DMSO, (-)-halofenic acid (75 and 150 µM) and then stimulated with 100 ng/mL LPS for 8 hours further. Following LPS stimulation cell supernatants were then harvested and stored at -200C prior to analysis of secreted cytokine levels using a ProcartaTM Cytokine Profiling Kit (Panomics Inc., Fremont, CA).

The cells were harvested in Qiazol lysis solution for gene expression analysis. RNA was isolated (using MagAttract RNA Universal Tissue M48 Kit as per manufacturer instructions), cDNA was prepared by reverse transcription (using High Capacity cDNA Reverse Transcription Kit as per manufacturer instructions) and RT-PCR (Taqman) was performed in 96 well PCR plates using a gene expression assay mix for IL-1β (ABI Cat#: Mm00434228_m1) and Taqman fast universal PCR master mix. The Ct values for IL-1β were determined using ABI Sequence Detection Software. The gene expression "Fold Change relative to LPS" was calculated using the comparative Ct method for relative quantitation as suggested by ABI (Foster City, CA). This method involved comparing the Ct values of the compound pre-treated samples + LPS to the vehicle treated samples + LPS. The Ct values of both the compound and vehicle treated samples were normalized to the endogenous housekeeping gene (GAPDH). This method is also known as the 2^{^}-[delta][delta]Ct method, where [delta] [delta]Ct = [delta]Ct(sample) - [delta]Ct(reference). Here, [delta]Ct(sample) is the Ct value for the compound treated sample normalized to the endogenous housekeeping gene and [delta]Ct(reference) is the Ct value for the vehicle treated sample normalized to the endogenous housekeeping gene. The fold change is then calculated using the formula 2^-[delta][delta]Ct. Each experimental condition was run in 4 replicate wells. The "Fold Change relative to LPS" data from the replicate experiments was pooled and significance was tested using 1-way ANOVA with Tukey post- hoc test (*= p<0.05, ** = p<0.01 and *** = p<0.001).

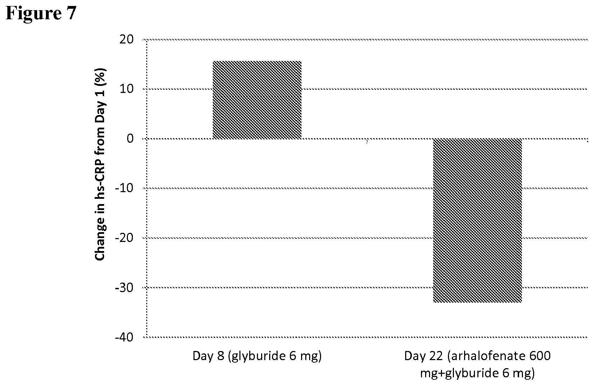

Data presented in FIGS. 6 and 7 were generated based on a single-center, Phase 1, randomized, single-blind, multiple-dose study (M102-0507) evaluating the effect of (-)-halofenate given to type 2 diabetic patients who were receiving a stable dose of Glynase® (micronized glyburide). Eligible patients were initially dosed for 3 days with Glynase® 3 mg/day, and if safety and tolerability were acceptable to the Investigator, the dose was increased to Glynase® 6 mg/day for 4 days, followed by 14 days of Glynase® 6 mg and either (-)-halofenate 400 mg or 600 mg administration until Day 21, the patients were discharged on Day 22. This schedule is summarized in Table 2.

**Table 2**

| | **Day 1- Day 3** | **Day 4- Day 7** | **Day 8- Day 21** | |
|---|---|---|---|---|
| | | | **Group 1 (400 mg)** | **Group 2 (600 mg)** |
| **Glynase®:** | 3 mg / day | 6 mg/day | 6 mg/day | 6 mg/day |
| (-)-halofenate | | | 400 mg/day | 600 mg/day |

In this study, change in high sensitivity C-Reactive Protein (hs-CRP) from baseline was one of the endpoints. It was observed that hs-CRP increased from Day 1 to Day 8 (20.8% in Group 1 and 15.6% in Group 2), and then declined between Days 8 and 22. At Day 22, the mean percentage changes in C-reactive protein compared to Day 1 were -21.1% and -32.9% in Group 1 ((-)-halofenate 400 mg) and Group 2 ((-)-halofenate 600 mg), respectively.

**Table 3**

| Treatment with (-)-halofenate 600 mg (from Day 8-22) | | | | | | | |
|---|---|---|---|---|---|---|---|
| Day 1 hs-CRP | Day 8 hs-CRP | Mean % change from Day 1 | Day 22 hs-CRP | Mean % change from Day 1 | p-value (vs. Day 1) | Mean % change from Day 8 | p-value (vs. Day 8) |
| 6.19 | 7.57 | 15.6 | 4.4 | -32.9 | 0.0091 | -33.3 | 0.0214 |

As shown in FIGS. 6-7 and Table 3, data from the M102-10507 study demonstrates that treatment with (-)-halofenate at a daily dose of 600 mg reduced the hs-CRP significantly from Day 1 (baseline) and from Day 8 (Glyburide only Phase). The mean hs-CRP changes were - 32.9% (p=0.0091) and -33.3% (p=0.0214), respectively.

### Examples

### Example 1: Suppression of Uric-Acid Induced Inflammation In Vitro

Differentiated murine 3T3-L1 adipocytes are cultured in vitro in 24 well plates. To the culture medium (-)-halofenic acid is added at a final concentration of 50- 150 µM prior to the addition of uric acid at 5 mg/dL or 15 mg/dL and the culture continued for 3 or 7 days. A parallel culture of cells is conducted in the presence of a vehicle such as dimethylsulfoxide (DMSO). At the end of the culture period media is removed, cells are isolated and messenger RNA prepared. The levels of secreted cytokines representing a panel of pro-inflammatory cytokines including but not restricted to monocyte chemoatractant protein-1 (MCP-1), tumor necrosis factor α (TNF-α), interlekin-1β, (IL-1β), interleukin-6 (IL-6) and interleukin-12 (Il-12) are determined in the media isolated from the cells using commercially available cytokine assay kits. The levels of gene expression for the mRNAs for a panel of pro-inflammatory cytokines including but not restricted to monocyte chemoatractant protein-1 (MCP-1), tumor necrosis factor α (TNF-α), interlekin-1β, (IL-1β), interleukin-6 (IL-6) and interleukin-12 (Il-12) are determined using real-time PCR. The addition of (-)-halofenate prevents the uptake of uric acid into 3T3-L1 adipocytes and thereby suppresses the uric acid induced inflammatory response resulting in a reduced level of expression and consequently secretion of this panel of pro-inflammatory cytokines. A similar study is also conducted in primary mouse macrophages and human umbilical vein endothelial cells.

### Example 2: Animal Gout Flare Model

The models described in R. Torres et al., Ann. Rheum. Dis. 68, 1602-08 (2009) (available at http://ard.bmj.com/content/68/10/1602.long), is used. Briefly, twenty C57BL6 mice are obtained from Jackson Laboratories (Bar Harbor ME USA) and used between the ages of 12 and 16 weeks. The mice are housed singly at least a week before study and allowed access to regular chow and water ad libitum. Arhalofenate is administered orally to half (ten) of the mice (test mice) daily at a dose of 125 mg/kg for periods of time including for example 1 day, 5 days and 2 weeks prior to induction of inflammation by uric acid. The remaining ten mice (control mice) are administered a vehicle consisting of 1 % Carboxymethyl Cellulose//2% Tween-80. In another treatment modality, arhalofenate is co-administered at the time of uric acid treatment.

Crystals of monosodium urate (MSU) are prepared as described in R. Liu-Bryan et al., Arthritis Rheum. 52, 2936-46 (2005). In one model, MSU crystals (0.5 mg) suspended in 20 microliters of endotoxin-free PBS are injected intro-articularly into the tibio-tarsal joint (ankle) of the mice anaesthetized with 2.5% isoflurane. Thermal hyperalgesia, weight bearing ability, angle joint diameter, and histological analyses are performed according to Torres et al, supra. The level of secretion of a panel of pro-inflammatory cytokines including but not restricted to monocyte chemoatractant protein-1 (MCP-1), tumor necrosis factor α (TNF-α), interlekin-1β, (IL-1β), interleukin-6 (IL-6) and interleukin-12 (Il-12) are measured in fluid isolated from the injected joints. Joints are dissected and homogenized to allow preparation of mRNA and the level of gene exrpression of the same panel of pro-inflammatory cytokines determined. In another model, mice are injected intraperitoneally with 1 mg of MSU suspended in 0.5 ml of endotoxin free PBS. After 6 h , mice are killed and their peritoneal cavities washed and harvested for measurement of neutrophil influx by staining with a neutrophil specific antibody R-phycoerythrin conjugated rat anti mouse Ly-6G monoclonal antibody. In another model, an air pouch is introduced subcutaneously and 1 mg of MSU is injected into the pouch. Six hours after crystal injection the cells resident in the pouch are collected by lavaging with 5 ml of buffer. Neutrophil infiltration is measured by staining as above. The levels of monocyte chemoatractant protein-1 (MCP-1), tumor necrosis factor α (TNF-α), interlekin-1β, (IL-1β), interleukin-6 (IL-6) and interleukin-12 (Il-12) are also measured in the lavaged fluid isolated from the urate injected air pouch.

### Example 3: Clinical Trial Comparing Effectiveness of Arhalofenate in the Reduction of Gout Flares During Initiation of and Maintenance Use of Therapy for Uric Acid Lowering

This is a double-blind, parallel group, multicenter, randomized study.

### Primary Endpoint

Proportion of patients experiencing an acute gout flare overall and in the separate timeframes of weeks 0-2 of treatment, weeks 3-12 of treatment, and weeks 11-12 of treatment.

### Secondary Endpoints:

a. Total number of gout flares
b. Duration of gout flares
c. Incidence of patient discontinuation due to gout flares.
d. Severity of gout flares

### Treatment Regime:

Individuals are randomized into three groups: a control group (n=100) and two experimental groups (n=100 each). Subjects in the control group are administered allopurinol (300 mg) once per day. Subjects in the experimental groups are administered arhalofenate (400 mg or 600 mg) tablets once per day. All patients receive flare prophylaxis for the first two weeks of treatment. Treatment duration with blinded study drug is three months.

### Inclusion Criteria:

Male or female, 18 years of age or older.

Female individuals of childbearing potential must have a negative pregnancy test. Female individuals of childbearing potential must be infertile or on contraception.

Serum uric acid > 6.0 mg/dL and < 12 mg/dL

Diagnosed with gout according to the 1980 ARA Criteria for the Classification of Acute Arthritis of Primary Gout.

### Analysis:

The primary analysis is based on a comparison between the control group and arhalofenate groups in the proportion of subjects experiencing a flare using an intent to treat approach. Pairwise comparisons between groups are made using Fisher's exact test.

While the foregoing description describes specific embodiments, those with ordinary skill in the art will appreciate that various modifications and alternatives can be developed. Accordingly, the particular embodiments and examples described above are meant to be illustrative only, and not to limit the scope of the invention, which is to be given the full breadth of the appended claims.

## Claims

1. A compound that is (-)-halofenate, or (-)-halofenic acid or a pharmaceutically acceptable salt thereof, substantially free from its (+)-enantiomer, for use in treating a gout flare or in reducing the number, frequency, duration, or intensity, of gout flares experienced by a subject during initiation or maintenance of therapy for uric acid lowering.

2. The compound for use according to claim 1 that is for treating a gout flare.

3. The compound for use according to claim 1 that is for reducing the number, frequency, duration, or intensity of gout flares.

4. The compound for use according to any one of claims 1 to 3 where the compound is (-)-halofenate.

5. The compound for use according to any one of claims 1 to 3 where the compound is (-)-halofenic acid or a pharmaceutically acceptable salt thereof.

6. The compound for use according to any one of claims 1 to 5 where the compound is administered orally.

7. The compound for use according to any one of claims 1 to 6 where the amount of the compound is effective for once/day dosing.

8. The compound for use according to any one of claims 1 to 7 where the compound is administered at 100 to 1000 mg/day, preferably at 400, 600, 800, or 1000 mg/day.

9. The compound for use according to any one of claims 1 to 8 where the compound is administered for four weeks or longer.

10. The compound for use according to any one of claims 1 to 9 where the subject is one being treated or requiring treatment with a flare prophylaxis agent or pain management agent.

11. The compound for use according to claim 10 where the flare prophylaxis agent or pain management agent is colchicine.

12. The compound for use according to any one of claims 1 to 9 where the subject is also administered a urate-lowering agent that is a xanthine oxidase inhibitor, an inhibitor of uric acid production, a uricosuric agent, or a uricase.

13. The compound for use according to claim 12 where the urate-lowering agent is allopurinol or febuxostat.

14. The compound for use according to claim 12 or 13 where the urate-lowering agent is febuxostat.

15. The compound for use according to claim 12 where the urate-lowering agent is probenecid, benzbromarone, or sulfinpyrazone.

## Patentansprüche

1. Verbindung, die (-)-Halofenat oder (-)-Halofensäure oder ein pharmazeutisch annehmbares Salz davon ist und im Wesentlichen frei vom jeweiligen (+)-Enantiomer ist, zur Verwendung zur Behandlung eines Gichtanfalls oder zur Reduktion der Anzahl, Häufigkeit, Dauer oder Intensität von Gichtanfällen bei einem Individuum während des Initiierens oder Weiterführens einer Therapie zur Senkung von Harnsäure.

2. Verbindung zur Verwendung nach Anspruch 1 zur Behandlung eines Gichtanfalls.

3. Verbindung zur Verwendung nach Anspruch 1 zur Reduktion der Anzahl, Häufigkeit, Dauer oder Intensität von Gichtanfällen.

4. Verbindung zur Verwendung nach einem der Ansprüche 1 bis 3, wobei die Verbindung (-)-Halofenat ist.

5. Verbindung zur Verwendung nach einem der Ansprüche 1 bis 3, wobei die Verbindung (-)-Halofensäure oder ein pharmazeutisch annehmbares Salz davon ist.

6. Verbindung zur Verwendung nach einem der Ansprüche 1 bis 5, wobei die Verbindung oral verabreicht wird.

7. Verbindung zur Verwendung nach einem der Ansprüche 1 bis 6, wobei die Menge der Verbindung für eine einmalige Dosierung/Tag wirksam ist.

8. Verbindung zur Verwendung nach einem der Ansprüche 1 bis 7, wobei die Verbindung in einer Menge von 100 bis 1.000 mg/Tag, vorzugsweise von 400, 600, 800 oder 1.000 mg/Tag, verabreicht wird.

9. Verbindung zur Verwendung nach einem der Ansprüche 1 bis 8, wobei die Verbindung vier Wochen lang oder länger verabreicht wird.

10. Verbindung zur Verwendung nach einem der Ansprüche 1 bis 9, wobei das Individuum ein Individuum ist, das mit einem Anfällen vorbeugenden Mittel oder einem Schmerz bekämpfenden Mittel behandelt wird oder einer Behandlung damit bedarf.

11. Verbindung zur Verwendung nach Anspruch 10, wobei das Anfällen vorbeugende Mittel oder Schmerz bekämpfende Mittel Colchicin ist.

12. Verbindung zur Verwendung nach einem der Ansprüche 1 bis 9, wobei dem Individuum auch ein Urat senkendes Mittel verabreicht wird, das ein Xanthinoxidasehemmer, ein Harnsäureproduktionshemmer, ein Urikosurikum oder eine Urikase ist.

13. Verbindung zur Verwendung nach Anspruch 12, wobei das Urat senkende Mittel Allopurinol oder Febuxostat ist.

14. Verbindung zur Verwendung nach Anspruch 12 oder 13, wobei das Urat senkende Mittel Febuxostat ist.

15. Verbindung zur Verwendung nach Anspruch 12, wobei das Urat senkende Mittel Probenecid, Benzbromaron oder Sulfinpyrazon ist.

## Revendications

1. Composé qui est le (-)-halofénate, ou l'acide (-)-halofénique ou un sel pharmaceutiquement acceptable de celui-ci, pratiquement exempt de son énantiomère (+), pour une utilisation dans le traitement d'une crise de goutte ou dans la réduction du nombre, de la fréquence, de la durée ou de l'intensité de crises de goutte subies par un sujet durant l'initiation ou la maintenance d'un traitement pour diminuer l'acide urique.

2. Composé pour une utilisation selon la revendication 1, qui est destiné à traiter une crise de goutte.

3. Composé pour une utilisation selon la revendication 1, qui est destiné à réduire le nombre, la fréquence, la durée ou l'intensité de crises de goutte.

4. Composé pour une utilisation selon l'une quelconque des revendications 1 à 3, lequel composé est le (-)-halofénate.

5. Composé pour une utilisation selon l'une quelconque des revendications 1 à 3, lequel composé est l'acide (-)-halofénique ou un sel pharmaceutiquement acceptable de celui-ci.

6. Composé pour une utilisation selon l'une quelconque des revendications 1 à 5, lequel composé est administré par voie orale.

7. Composé pour une utilisation selon l'une quelconque des revendications 1 à 6, dans lequel la quantité du composé est efficace pour une administration une fois par jour.

8. Composé pour une utilisation selon l'une quelconque des revendications 1 à 7, lequel composé est administré à raison de 100 à 1000 mg/jour, de préférence à raison de 400, 600, 800 ou 1000 mg/jour.

9. Composé pour une utilisation selon l'une quelconque des revendications 1 à 8, lequel composé est administré pendant quatre semaines ou plus longtemps.

10. Composé pour une utilisation selon l'une quelconque des revendications 1 à 9, dans lequel le sujet est en cours de traitement ou requiert un traitement avec un agent de prophylaxie des crises ou un agent de gestion de la douleur.

11. Composé pour une utilisation selon la revendication 10, dans lequel l'agent de prophylaxie des crises ou l'agent de gestion de la douleur est la colchicine.

12. Composé pour une utilisation selon l'une quelconque des revendications 1 à 9, dans lequel il est également administré au sujet un agent diminuant l'urate qui est un inhibiteur de xanthine oxydase, un inhibiteur de production d'acide urique, un agent uricosurique, ou une uricase.

13. Composé pour une utilisation selon la revendication 12, dans lequel l'agent diminuant l'urate est l'allopurinol ou le fébuxostat.

14. Composé pour une utilisation selon la revendication 12 ou 13, dans lequel l'agent diminuant l'urate est le fébuxostat.

15. Composé pour une utilisation selon la revendication 12, dans lequel l'agent diminuant l'urate est le probénécid, la benzbromarone, ou la sulfinpyrazone.
